# EUROPEAN PATENT APPLICATION

(11) **EP 3 023 790 A1**
(43) Date of publication of application: **25.05.2016**
(21) Application number: 15198256.8
(22) Date of filing: 12.12.2012
(51) Int. Cl.: G01N 33/569

(54) **ASSAY FOR INFLUENZA VIRUS HEMAGGLUTININS**

(30) Priority: 12.12.2011 US 201161569597 P
(62) Divisional of application: 12818607.9
(71) Applicant: Novartis AG, 4056 Basel (CH)
(72) Inventor: DORMITZER, Philip, Cambridge, MA 02139 (US); ANDREWS, William, Cambridge, MA 02139 (US); RINELLA, Paola, 53100 Siena (IT); ROSA, Domenico, 53100 Siena (IT); PALLADINO, Giuseppe, Cambridge, MA 02139 (US)
(74) Representative: Carpmaels & Ransford LLP

(57) **Abstract**

The invention quantifies HA using a process which comprises two key steps: HA is captured using a lectin, such as snowdrop lectin; and captured HA is quantified using an immunoassay. This assay can conveniently be performed in an ELISA format. The process is specific, is robust in the face of contaminants, and is more rapid and more sensitive than the standard SRID assay.

## Description

This patent application claims the benefit of United States provisional application 61/569,597, filed December 12th 2011, the contents of which are hereby incorporated herein in full.

### TECHNICAL FIELD

This invention is in the field of assays for influenza virus hemagglutinin *e.g.* for analysing vaccines.

### BACKGROUND ART

The standard assay for hemagglutinin (HA) content in inactivated influenza vaccines is based on single radial immunodiffusion ("SRID") [1,2], which was recommended by the WHO in 1978 to replace tests based on agglutination of erythrocytes.

Although the SRID assay is well established, it is slow to perform, has poor dynamic range, considerable variability, and it can take a long time to prepare and calibrate the required specific anti-HA serum. Moreover, it cannot be used for in-process monitoring. Thus people have looked for non-SRID assays for quantifying influenza HA.

For instance, reference 3 discloses an assay which uses ultrafiltration followed by RP-HPLC. Reference 4 discloses a method where HA is reduced in the presence of a detergent, alkylated to protect its sulfhydryl groups, applied to a RP-HPLC column, and then eluted with an ion pairing agent in an organic mobile phase. Reference 5 uses electrophoretic separation of deglycosylated samples in conjunction with densitometry. ELISA assays have also been reported *e*.*g*. reference 6.

Some alternatives to SRID involve modification of the samples (*e.g.* deglycosylation, denaturation, or sulfhydryl derivatisation). Some also lack strain discrimination, so they are not useful for analysis of products containing multiple different hemagglutinins (*e.g.* seasonal vaccines, which usually contain HA from 3 viral strains). Furthermore, approaches based on some physicochemical techniques can require highly-trained staff.

There remains a need for further and improved alternatives to the SRID assays for quantifying influenza virus HA.

### DISCLOSURE OF THE INVENTION

The invention quantifies HA using a method which comprises two key steps: HA is captured using a lectin, such as snowdrop lectin; and captured HA is quantified using an immunoassay. This assay can conveniently be performed in an ELISA format. The method is specific, is robust in the face of other components in the sample, and is more rapid and more sensitive than the current SRID assay. Conveniently, however, it can utilise the reagents (*e.g*. anti-HA antibodies) which are currently prepared by public health authorities for use in the SRID assay, and can be used to quantify different HA proteins in a single captured sample by using different anti-HA antibodies for the immunoassay.

Thus the invention provides a method for detecting influenza virus hemagglutinin in a sample (*e.g.* in a vaccine), comprising steps of: (i) contacting the sample with a lectin which is immobilised on a solid support, to provide captured hemagglutinin; and (ii) detecting the captured hemagglutinin by immunoassay. The detection step is ideally a quantitative step, thereby permitting the amount of HA in the original sample to be quantified. Thus the invention also provides a method for quantifying the amount and/or concentration of influenza virus hemagglutinin in a sample *(e.g.* in a vaccine), comprising steps of: (i) contacting the sample with a lectin which is immobilised on a solid support, to provide captured hemagglutinin; and (ii) detecting the captured hemagglutinin by quantitative immunoassay. The amount of HA detected in step (ii) can be used to calculate the amount and/or concentration of HA in the sample.

The invention also provides bulk antigen containing influenza HA, wherein a sample of the bulk material has been analysed by a method of the invention.

The invention also provides a vaccine comprising influenza HA, wherein the vaccine is (i) a vaccine prepared from a bulk antigen which has been analysed by a method of the invention and/or (ii) part of a batch of vaccines in which at least one vaccine from the batch has been analysed by a method of the invention.

The invention also provides, in a method for analysing a sample's influenza virus HA content by ELISA, the improvement consisting of using a lectin to capture the HA.

The invention also provides an ELISA kit comprising (i) a multi-well solid support (ii) a lectin and (iii) an enzymatic HA detection reagent. The kit can also include a substrate for the enzymatic HA detection reagent. The detection reagent can be an enzyme-conjugated anti-HA antibody or a tagged anti-HA antibody to which an enzyme can be attached. The lectin in the kit can be in free form but is ideally immobilised to the solid support *i.e.* the kit comprises (i) a multi-well solid support, in at least one well of which a lectin is immobilised for capture of influenza virus HA and (ii) an enzymatic HA detection reagent.

The invention also provides a solid support to which a lectin (*e*.*g*. GNA lectin) is immobilised, for use in ELISA analysis of influenza virus HA, particularly for analysis of an influenza vaccine comprising HA. Such solid supports are known in the art with GNA lectin [7] but have not previously been used for influenza HA or vaccine analysis. The support can be a multi-well plate. The support is advantageous because its binding properties mean that it can be used to analyse HA from a wide range of different influenza virus strains *i.e.* it can be used as a universal influenza virus HA capture reagent.

The invention also provides an antibody specific for an influenza virus hemagglutinin, wherein the antibody has a biotin or streptavidin group for attachment of an enzyme for performing an ELISA. The antibody is ideally a sheep antibody *e.g.* a polyclonal sheep serum as currently used for SRID assays.

The lectin-based capture step is useful for purification of HA even without any detection or quantification. Thus, the invention also provides a method for purifying influenza virus hemagglutinin in a sample, comprising a step of contacting the sample with a lectin which is immobilised on a solid support. HA captured by the lectin can then be eluted to provide purified HA material. Thus the lectin-based capture step can be used as a preparative technique for influenza HA *e.g.* to prepare HA standards with high purity. Such standards can be useful in the preparation of influenza vaccines.

The invention also provides an affinity chromatography support or column to which a lectin (*e.g.* GNA lectin) is immobilised, for use in affinity purification of influenza virus HA. Such materials are already available (*e.g.* agarose-linked GNA lectin from Vector Laboratories) but their use in purification of influenza virus HA is new.

The invention also provides a purified influenza virus HA which has been prepared by a method involving capture of the HA by a lectin. The purified HA preferably has a known HA concentration such that it can be used as a standard reagent *e.g.* as part of vaccine manufacture. This purified HA is particularly useful when prepared from influenza viruses grown in cell culture *e.g.* in MDCK cells. The invention also provides a method for preparing an influenza vaccine, comprising a step of analysing the vaccine (or a sample thereof) by using a HA standard prepared by this method. For instance, the purified HA can be used as a control or as a calibration reagent for other assays *e.g.* for SRTD. Usefully, the purified HA is substantially free from other proteins (*e*.*g*. from influenza virus neuraminidase) such that HA is substantially the only protein component in the material.

### Lectin capture of HA

Methods of the invention use an immobilised lectin to capture influenza virus HA. The lectin ideally binds to mannose residues, such as α-1,3-linked mannose residues. Various mannose-binding lectins are known in the art, and these are typically plant lectins (and, in particular, monocot lectins) such as those from snowdrop (*Galanthus nivalis*, 'GNA' lectin), from daffodil (*Narcissus pseudonarcissis*, 'NPA' lectin), from garlic (*Allium sativum*, 'ASA' lectin), and from lentil (*Lens culinaris*, 'LCH' lectin). Concanavalin A (from *Cancavella ensiformis*, 'ConA' lectin) is a commonly-used mannose-binding lectin. The lectin for use with the invention is preferably a mannose-binding lectin, but not a ConA lectin. The lectin is preferably not a galactose-binding lectin. Examples of galactose-binding lectins include the β-galactose specific *Erythrina christagalli* lectin (ECL) and the α-galactose specific *Euonymus europaeus* lectin (EEL).

Mannose is a common carbohydrate residue in glycoproteins, *e.g.* HA and NA. It was unclear whether mannose-binding lectins would be able to specifically and efficiently capture HA from other viral components. Surprisingly, the inventors found that mannose-binding lectin (*e.g.* GNA lectin) is able to capture HA with high specificity. In particular, the mannose-binding lectin is able to capture HA of different influenza virus strains, independent of their HA glycosylation levels. The inventors also found that mannose-binding lectin is also able to capture HA of influenza viruses that had been grown in various hosts, *e*.*g*. eggs and MDCK cells. Hence, variation of host cell dependent glycosylation of viral envelope glycoproteins does not appear to affect the capture specificity of mannose-binding lectin to HA.

The lectin for use with the invention ideally has a higher binding affinity to HA than to other viral envelope proteins (*i.e.* not HA), empty capsids, nucleic acids and/or proteins from hosts (*i.e.* for growing the influenza virus). The preferred lectin for use with the invention is GNA, originally prepared from snowdrop bulbs. This lectin naturally is a homotetramer and at least six different amino acid sequence variants or isoforms have been reported for GNA lectin (SEQ ID NOs: 1 to 6 herein). Its 3D structure has been determined. Each monomer in the tetramer has three sub-domains, each of which can bind to a mannose to give a 12-valent lectin. Unlike many mannose-binding lectins (a) it is not a metalloprotein and its binding activity does not require bivalent cations (b) it does not also bind to α-linked glucose residues. As shown below, GNA lectin efficiently captures influenza virus HA to provide purified material *e*.*g*. for detection, for quantification, or for use as a standard antigen, *etc.*

The invention can use a wild-type GNA lectin or can use modified forms which still retain the useful HA-binding activity of wild-type GNA lectin. Thus the lectin used with the invention can comprise any of amino acid sequences SEQ ID NOs: 1 to 6, which are variants of the GNA lectin precursor. As an alternative, the lectin can (i) comprise a fragment of at least *n* consecutive amino acids from any of SEQ ID NOs: 1 to 6; and/or (ii) comprise an amino acid sequence with at least *x%* sequence identity to any of amino acid sequences SEQ ID NOs: 1 to 6, provided that it can bind to influenza virus HA, where x is 70 or more *e.g.* 75, 80, 85, 88, 90, 92, 94, 95, 96, 97, 98, 99 or more (*e.g*. 100) and n is 7 or more (*e.g.* 8, 10, 12, 14, 16, 18, 20, 25, 30, 35, 40, 50, 60, 70, 80, 90 or more). Suitable fragments include those lacking the native signal peptide of SEQ ID NOs: 1 to 6 (*e.g*. lacking amino acids 1-26 of SEQ ID NO: 6).Various GNA-related lectins are known in the art [8]. However, none of the known GNA, variants or isoforms thereof, and GNA-related lectins has been used as part of influenza vaccine manufacture, *e.g.* for purifying, detecting or quantifying HA.

The lectin will typically be used in the form of an oligomer *e.g.* a tetramer.

For use with the invention the lectin is immobilised on a solid support. Appropriate supports can be chosen depending on the intended format of any assay *e.g.* the support might be a solid plate, a bead, a column support, *etc.* A preferred support is a plate suitable for use in an ELISA assay. For instance, the lectin can be immobilised on a plastic multiwell plate *e.g.* a 96-well or 384-well microtitre plate. A polystyrene support is typical for ELISA, although polylactate supports can also be used.

Other solid supports, *e*.*g*. those typically used in affinity chromatographic methods, include packed beds, membrane adsorbers and monoliths [9]. For packed beds, the matrices typically contain resins (beads) and are usually made of polymer or inorganic resin materials. Polymer-based beads can be made of natural polymers (*e.g.* agarose, agarose-dextran composites, cellulose and dextran) or synthetic polymers (*e.g.* polyacrylamide, polystyrene divinylbenzene, derivatives of polyacrylamide and polymethacrylate). Examples of inorganic resin materials include hydroxyapatite, silica and controlled-pore glass. Beads can be spherical and non-spherical shaped and are available as porous, nonporous and solid-core resins. Pore sizes of conventional porous beads range from 10 to 100 nm. Monoliths include those made of polymethacrylate copolymers, polyacrylamide, polystyrene, polystyrene-divinylbenzene, modified cellulose and silica. Monoliths can be disk-, tube- and rod-shaped with various pore sizes. Suitable membrane adsorbers include cellulose, polyamine, polysulfone, hydrazide and composite membranes (*e.g.* polyethylene oxide and polyethersulfone coated with hydroxyethyl-cellulose. The membrane adsorbers can be flat sheets, hollow fibres or radial-flow devices.

Immobilisation of the lectin can be achieved in various ways. For many assay formats (*e.g.* ELISA) it can be sufficient to adsorb the protein non-specifically to a support; for other formats the lectin can be immobilised by means of a covalent linker using standard chemistry *e.g.* as commonly used for preparing affinity chromatography supports.

For embodiments where captured HA will be assayed it is not necessary to elute the HA before it is assayed (but for some non-ELISA assay formats such elution might nevertheless be desired or necessary). For embodiments where HA is being purified then the HA must be eluted after capture, and this can be achieved by contacting the immobilised lectin with a high affinity ligand for the lectin *e.g.* with free mannose or mannose-containing oligosaccharide, as typical in affinity chromatography.

### Immunoassay of HA

Most embodiments of the invention include a step in which captured HA is detected using an immunoassay. The immunoassay is ideally a quantitative immunoassay, such that the detection step permits the amount of HA in the sample to be determined.

The immunoassay will use an anti-HA antibody, which can be polyclonal or monoclonal. Ideally the antibody is strain-specific, such that a particular HA can be detected even in the presence of other HA species. For instance, in a current seasonal vaccine (A/H1N1, A/H3N2, and B strains) the invention (like SRID) can permit the measurement of each of the three HA proteins even in the presence of each other by using three different anti-HA antibodies, each of which is specific for only one of the HA proteins. Thus the invention can involve universal capture (via a lectin which binds to each HA) but specific detection (via an immunoassay which can distinguish between different HA proteins which are present in the sample). In some embodiments, however, particularly those for analysing monovalent HA materials (*e.g.* monovalent bulk vaccines) the invention can readily be performed using an anti-HA antibody (*e.g.* a monoclonal antibody) that is broadly reactive with HA from multiple HA types or with HA from multiple strains with the same HA type.

Suitable anti-HA antibodies are widely available. For example, public health agencies prepare antisera for use in vaccine analysis in each influenza season. These antisera can be used with the invention, but other anti-HA antibodies can also be used. The antibody may be modified to permit attachment of an enzyme for ELISA purposes (see below).

The anti-HA antibody is ideally an IgG antibody. Sheep IgG is particularly useful with the invention. Suitable sheep IgG can be purified from reference sera provided as SRID reagents by public health authorities.

The immunoassay is ideally performed *in situ*, that is with HA which is still bound to lectin. In another embodiment, the HA is eluted from the lectin and the immunoassay is performed post-elution, *i.e.* HA is no longer bound to lectin.

### ELISA format

A preferred embodiment of the invention is an ELISA format, and more preferably a sandwich ELISA format. This format is useful because reagents, equipment and technicians are very familiar with it, and standard protocols (*e.g.* immobilisation, capture, washing, detection, calibration, *etc*.) can easily be used to provide reliable quantitative results.

In the ELISA format the immobilised lectin captures HA, and the captured HA is then exposed to anti-HA antibodies. The assay format can involve a primary antibody (which binds to the HA) and a secondary antibody (which binds to the primary antibody), with the secondary antibody comprising an enzyme. This primary/secondary arrangement means that the same enzyme-linked secondary antibody can be used multiple times, without needing to prepare a new enzyme-conjugated detection antibody for each new HA. In a preferred format, however, the ELISA uses a tagged anti-HA antibody, which is then contacted with an enzyme which binds to the tag. For example, a biotin-conjugated anti-HA antibody can bind to captured HA and then a streptavidin-conjugated enzyme is added. The enzyme which is now immobilised on the solid support is then used to catalyse a reaction which can easily be assayed, as in the standard ELISA technique.

The invention can use any enzyme which is suitable for use in ELISA *e.g.* any enzyme which can convert a substrate into a detectable product, such as a coloured, luminescent (*e.g.* fluorescent) or electrochemically-active product. Such enzymes include, but are not limited to, alkaline phosphatase (which can convert pNPP to a coloured soluble product) or a peroxidase such as horseradish peroxidase (which can convert tetramethyl benzidine to produce a coloured soluble product; other typical substrates include ABTS, OPD).

The invention utilises standard and known steps from existing ELISA protocols *e.g.* calibration, washing, negative controls, positive controls, replication, *etc.* The ELISA technique is ideally performed in a 96- or 384-well plate.

In some embodiments the invention can utilise the alternative ELISA protocol disclosed in reference 10. In this protocol the anti-HA antibody and the HA are mixed before contacting the HA with the lectin. In other embodiments, however, the standard ELISA protocol is used, in which the HA is captured by the lectin and then the anti-HA antibody is added. If the alternative protocol is used then the method should uses an anti-HA antibody which does not block the lectin's binding site on the HA, and it is straightforward to screen for monoclonal antibodies which meet this requirement.

The inventors found that ELISA can be sensitive to anti-host (*e.g.* eggs) antibodies, *e.g.* which can be found in NIBSC and CBER reference sera, and this could lead to inaccurate calibration and/or inaccurate quantification of a sample. The inventors found that this inaccuracy can be minimised if the virus to which anti-HA antibodies are raised and the HA (in the calibration standards or samples) are derived from different sources. For example, anti-HA raised against influenza viruses that had been grown in eggs (*i.e*. egg-derived influenza viruses) can be used to detect HA from influenza viruses that had been grown in cell culture (*i.e.* cell culture-derived HA). Conversely, anti-HA antibodies raised against influenza viruses that had been grown in cell culture (*i.e.* cell culture-derived influenza viruses) can be used to detect HA from influenza viruses that had been grown in eggs (*i.e.* egg-derived HA).

Thus, the invention provides a method for quantifying the HA in a sample comprising cell culture-derived HA using the ELISA format of the invention, wherein the ELISA is calibrated using cell culture-derived HA and anti-HA antibodies raised against egg-derived influenza viruses. Useful anti-HA antisera include those provided byNIBSC or CBER.

Conversely, the invention provides a method for quantifying the HA in a sample comprising egg-derived HA using the ELISA format of the invention, wherein the ELISA is calibrated using egg-derived HA and anti-HA antibodies raised against cell culture-derived influenza viruses.

The antigen calibration standard for the ELISA typically has a known HA concentration, *e.g.* as determined by ELISA, SRID or other conventional assays such as absorption at 280nm and BCA assay.

### Purification of HA

The methods of the invention use lectin in a capture step to purify HA from a sample. Thus, the invention provides a method for purifying influenza virus HA in a sample, comprising a step of contacting a sample with a lectin which is immobilised on a solid support. For example, the invention uses lectin as the affinity ligand in affinity chromatographic methods. In one embodiment, the invention uses lectin immobilised on an affinity chromatography support or column for recovery and purification of HA from a sample.

Ideally, the purified HA protein retains its immunogenicity (*i.e*. it can elicit antibody production when administered in a subject) and activity (*e.g.* it can agglutinate erythrocytes). The purified HA can be used as a vaccine component. Thus, the invention provides a method for preparing an influenza virus vaccine comprising HA, wherein the method comprises: contacting a sample with a lectin which is immobilised on a solid support, and purifying HA from the sample. The invention also provides an influenza virus vaccine comprising HA purified by the methods of the invention.

Alternatively, the purified HA can be used as a control or as a calibration standard, *e.g.* in immunoassays (*e*.*g*. ELISA or SRID), for the preparation of influenza vaccines. Thus, the invention provides a method for preparing a HA calibration standard, comprising contacting a sample with a lectin which is immobilised on a solid support, and purifying HA from the sample. The invention also provides a HA calibration standard prepared according to the methods of the invention.

The methods of the invention can use a mannose-binding lectin, with the proviso that the lectin is not a ConA lectin. Preferably, the lectin is a GNA lectin, a variant or isoform thereof, or a GNA-related lectin, as discussed above.

The methods of the invention can use a sample comprising antigens from influenza viruses that had been grown in eggs or mammalian cells. Preferably, the mammalian are not MDCK or Vero cells. The methods of the invention can use a sample comprising antigens from at least one influenza virus strain. Preferably, the sample comprises more than one influenza virus strains.

The methods of the invention can comprise treating a sample comprising influenza virus prior to purifying HA, *e.g.* inactivating and/or splitting the influenza virus. Thus, a method for purifying HA can use a sample comprising inactivated viruses, including whole virons, split virions, purified surface antigens or virosomes. Preferably, the sample does not contain whole virions. Preferably, the sample does not contain whole virions of strains A/Puerto Rico/8/34, A/Wisconsin/67/2005 and B/Malaysia/2506/2004.

Alternatively, the methods of the invention can use a sample comprising live influenza viruses. The invention also provides an influenza virus vaccine comprising HA, wherein the vaccine is (i) a vaccine prepared from a HA bulk, wherein the HA in the bulk has been quantified in a method that uses a HA calibration standard prepared according to the methods of the invention, and/or (ii) part of a batch of vaccines in which at least one vaccine from the batch has been quantified in a method that uses a HA calibration standard prepared according to the methods of the invention.

The purified HA sample is ideally substantially free from other viral components, such as free from neuraminidase (NA), free viral envelope proteins, empty capsids and nucleic acids. The purified HA is also substantially free from host (*e.g.* egg or cell) proteins, nucleic acids and reagents from cell culture (if viruses were grown in cell culture). Ideally, the purified sample has minimal host (*e.g.* egg or cell such as MDCK cell) proteins.

Ideally, the invention uses mannose-binding lectin as the affinity ligand in affinity chromatographic methods that are known in the art for purifying proteins, *e.g.* spin columns or FPLC [*e.g.* References 11,12]. Chromatography purification steps typically include incubating a sample with the affinity ligand to capture the analyte, and eluting the captured analyte. Optional wash steps can be included.

For example, the purification methods of the invention may include equilibrating a solid support on which lectin is immobilised (*e.g.* an affinity chromatography support or column), adsorbing lectin on the support, incubating a sample with the immobilised lectin to capture HA, and eluting the captured HA as purified HA proteins. Optionally, the sample can be mixed with octylglucoside (*i.e.* n-Octyl-beta-D-glucopyranoside) prior to incubating with the immobilised lectin. For example, octylglucoside is present at a final concentration of 1.5% (w/v).

Solid supports useful for the invention arc discussed above, and arc also discussed in reference 13. Suitable supports include polymer matrices, *e.g.* those having a mean pore diameter of about 100 nm and ligand density between 4.0 and 5.0 mg/ml. Lectin immobilized on polymer matrices can be purchased from GALAB Technologies GmbH. Supports containing agarose, *e.g.* Crosslinked Sepharose 4B, is also suitable for the invention, particularly those with a ligand density of about 3 mg/ml of settled gel. Lectin immobilised on crosslinked sepharose are commercially available, *e.g.* from Vector Laboratories Inc. Actigel ALD (Sterogene Bioseparation Inc.) with a bead size of about 60-160 µm and about 4% agarose may also be suitable.

Suitable solid supports containing activated porous glass particles may also be useful, *e.g.* Trisopor® 4000 Diol from VitraBio GmbH, which has a particle size from about 125 to 200 µm and a pore size of about 350-400 nm. Other useful solid supports include those containing cellulose beads, *e.g.* Cellufme™ Formyl (Chisso Corporation) having a particle size of about 125-210 µm. Reinforced cellulose membranes are also suitable, *e.g.* Sartobind® Epoxy membrane adsorbers (Sartorius AG) having pore size *e.g.* about 0.45 µm.

Immobilisation of the mannose-binding lectin can be achieved in various ways. For example, the mannose-binding lectin can be immobilised by means of a covalent linker using standard chemistry *e.g.* as commonly used for preparing affinity chromatography supports. Typically, the immobilised lectin density is about 3-8 mg/ml adsorbent. The skilled person would be able to determine the appropriate immobilised lectin density according to known techniques in the art.

Elution of the captured HA may be achieved by contacting the immobilised lectin with a high affinity ligand for the lectin *e.g.* with free mannose or mannose-containing oligosaccharide, as typical in affinity chromatography. The captured HA can be eluted using α-methyl-mannopyranoside.

Preferably, at least 75%, 80%, 90% or 95% of the HA (mass) is recovered from the total HA (mass) in the starting material using purification methods of the invention.

The HA in the purified sampled can be quantified, *e.g.* as determined by ELISA of the invention or other assays, *e.g.* ELISAs that use antibodies to capture HA, SRID, absorption at 280nm and BCA assay.

The purified HA may be treated by one or more additional steps, *e.g.* size exclusion chromatography, ion-exchange chromatography, HPLC, chromatography using heparinized or sulphated matrices, *etc.* The methods of the invention may include further tests commonly used in the art to assess immunogenicity, activity or toxicity of the purified HA.

When the purified HA is used as a vaccine, the vaccine ideally does not contain lectin. In embodiments where the purified HA sample contains lectin, additional steps can be included to remove the lectin. Vaccines containing <10ng (*e.g.* <1ng, <100pg) host cell DNA per 15µg of hemagglutinin are preferred, as are vaccines containing <10ng (*e.g.* <lng, <100pg) host cell DNA per 0.25ml volume. Vaccines containing <10ng (*e.g.* <1ng, <100pg) host cell DNA per 50µg of hemagglutinin are more preferred, as are vaccines containing <10ng (*e.g.* <1ng, <100pg) host cell DNA per 0.5ml volume.

### The sample

The sample which is analysed or purified using the invention contains (or is at least suspected to contain) influenza virus hemagglutinin (HA). SRID is often performed on material containing HA from several strains (*e.g.* trivalent material). The invention can be used with multivalent samples (*e.g.* 2-valent, 3-valent, 4-valent or more) and can separately detect and/or quantify the antigen from each strain by using appropriate detection reagents, which are already known for use with SRID. In other embodiments, however, the invention can be used to analyse or purify HA in monovalent samples.

The invention can be used with various types of sample. It can be used to analyse a final vaccine, but as the analytical method is destructive this will usually be a single vaccine from a batch, with analytical results indicating properties of the batch. The sample may have been taken from bulk vaccine material, with analytical results indicating properties of the bulk as a whole. In other embodiments the sample may even be virus-containing fluids *e.g.* it may be virus-containing harvest fluids from a cell culture or from eggs. The sample may thus be starting material, final vaccine material, or any manufacturing intermediate between virus culture and final vaccine. Preferably, the sample has minimal proteins from the host (*i.e.* the host for growing the influenza virus).

The sample will typically include HA from influenza virions but, as an alternative, it may include HA which was expressed in a recombinant host (*e.g.* in an insect cell line using a baculovirus vector) and purified [14,15,16] or may be in the form of virus-like particles (VLPs; *e.g.* see references 17 and 18). In general, however, antigens will be from virions and so the sample may, in addition to HA, include other influenza virus proteins (*e.g*. PB1, PB2, PA, NP, NA, M1, M2, NS1 and/or NS2 proteins), and may also include influenza virus lipids.

Virion-derived influenza virus antigens are based either on live virus or on inactivated virus (*e*.*g*. see chapters 17 & 18 of reference 19). Although the invention can be used to determine HA levels from live virus, dosing of live vaccines is based on median tissue culture infectious dose rather than HA content, and so the invention will usually be used to determine HA levels in an inactivated vaccine.

Inactivated vaccines may be based on whole virions, 'split' virions, purified surface antigens (including HA and, usually, also including neuraminidase) or virosomes (nucleic acid free viral-like liposomal particles [20]). The invention can be used with all such vaccines. The BEGRIVAC™, FLUARIX™, PREPANDRIX™, FLUZONE™ and FLUSHIELD™ products are split vaccines. The FLUVIRIN™, AGRIPPAL™, FLUAD™ and INFLUVAC™ products are surface antigen vaccines. The INFLEXAL V™ and INVAVAC™ products are virosome vaccines. The invention is most useful for measuring HA content in split and surface antigen vaccines. Thus, where the text refers to capture of HA, this can be capture of free HA, or can be capture of HA in combination with other components of influenza virus *e.g.* HA as part of an influenza virion. Similarly, where the text refers to detection of HA, this can be detection of free HA, or can be detection of HA in combination with other components of influenza virus *e.g.* detection of HA as part of an influenza virion. In preparative purification methods of the invention, however, the HA is ideally prepared as purified HA protein *e.g.* which is substantially free from other viral components, such as free from neuraminidase.

The invention can be used with HA from any influenza virus type, including both influenza A virus and influenza B virus (irrespective of origin, *e.g.* human, equine, avian, canine, swine flu). The invention is therefore suitable for analysing or purifying HA from a variety of influenza strains. The invention is preferably used for human influenza viruses, particularly strains for inclusion in human vaccines.

For influenza A viruses the invention can be used with HA from any known HA subtype (H1, H2, H3, H4, H5, H6, H7, H8, H9, H10, H11, H12, H13, H14, H15 or H16), and it is particularly useful with H1, H3 and H5 strains. The strain may have any of NA subtypes N1, N2, N3, N4, N5, N6, N7, N8 or N9. For example, the invention can be used to analyse material from a H1N1 strain, a H1N2 strain, a H3N2 strain, a H5N1 strain, *etc.* The invention is very useful for analysing trivalent vaccines *e.g.* containing HA from each of a A/H1, A/H3 and B virus.

For influenza B viruses the invention can be used with HA from a B/Victoria/2/87-like strain or a B/Yamagata/16/88-like strain. These two groups of strains are usually distinguished antigenically, but differences in amino acid sequences have also been described for distinguishing the two lineages *e.g.* B/Yamagata/16/88-like strains often (but not always) have HA proteins with deletions at amino acid residue 164, numbered relative to the 'Lee40' HA sequence [21].

HA which is analysed by methods of the invention may be full-length precursor HA, known as HA0. In some embodiments, however, the HA is a fragment of HA0. HA0 can be fragmented by serine proteases, such as trypsin, to yield a N-terminal fragment (HA1) and a C-terminal fragment (HA2), which can remain joined by a disulfide bridge. Thus the methods of the invention may purify HA0, HA1 or HA2, and may involve detection and analysis of only one such fragment (*e.g.* of HA1). Other HA fragments which might be purified and analysed include, for instance, the fragment released by bromelain treatment, or fragments obtained by treatment with both bromelain and trypsin Typically the methods will purify HA1, and thus at least the RP-HPLC step should be performed under reducing conditions (*e.g.* using DTT) to ensure HA1 and HA2 are separated, and optionally may involve a pre-purification digestion step (*e.g.* using trypsin) to ensure full cleavage of HA1 from HA2 (but this digestion is often unnecessary, and the presence of any full-length HA0 can easily be tested to determine if digestion would be useful).

HA in the sample may include a hyper-basic region around the HA1/HA2 cleavage site, but in other embodiments this region is absent.

HA in the sample may have a binding preference for oligosaccharides with a Sia(α2,6)Gal terminal disaccharide compared to oligosaccharides with a Sia(α2,3)Gal terminal disaccharide, or *vice versa*, or it may show no such preference. Assays for this preference are discussed in reference 22. Human influenza viruses bind to receptor oligosaccharides having a Sia(α2,6)Gal terminal disaccharide (sialic acid linked α-2,6 to galactose), but eggs and Vero cells have receptor oligosaccharides with a Sia(α2,3)Gal terminal disaccharide.

In some embodiments the HA has a different glycosylation pattern from the patterns seen in egg-derived viruses. Thus the HA may include glycoforms that are not seen in chicken eggs. Useful HA includes canine glycoforms *e*.*g*. the virus may have been grown in MDCK cells. Other useful HA includes human glycoforms *e.g.* the virus may have been grown in PER.C6 cells. Other useful HA includes simian glycoforms *e.g.* the virus may have been grown in Vero cells. These cell lines are widely available *e.g.* from the American Type Cell Culture (ATCC) collection, from the Coriell Cell Repositories, or from the European Collection of Cell Cultures (ECACC). For example, the ATCC supplies various different Vero cells under catalog numbers CCL-81, CCL-81.2, CRL-1586 and CRL-1587, and it supplies MDCK cells under catalog number CCL-34. PER.C6 is available from the ECACC under deposit number 96022940. One suitable MDCK cell line is 'MDCK 33016', deposited as DSM ACC 2219 [23].

The most preferred cell lines for growing influenza viruses are MDCK cell lines [23-26], derived from Madin Darby canine kidney. The original MDCK cell line is available from the ATCC as CCL-34, but derivatives of this cell line may also be used. For instance, reference 23 discloses a MDCK cell line that was adapted for growth in suspension culture ('MDCK 33016', deposited as DSM ACC 2219). Similarly, reference 27 discloses a MDCK-derived cell line that grows in suspension in serum-free culture ('B-702', deposited as FERM BP-7449). Reference 28 discloses non-tumorigenic MDCK cells, including 'MDCK-S' (ATCC PTA-6500), 'MDCK-SF101' (ATCC PTA-6501), 'MDCK-SF102' (ATCC PTA-6502) and 'MDCK-SF103' (PTA-6503). Reference 29 discloses MDCK cell lines with high susceptibility to infection, including 'MDCK.5F1' cells (ATCC CRL-12042). Any of these MDCK cell lines can be used.

The inventors found that mannose-binding lectin is able to specifically capture HA of influenza viruses that had been grown in various hosts, e.g. eggs and MDCK cells. Thus, the methods of the invention are able to capture HA independent of their HA glycosylation profiles. HA in the sample may be from a wild-type virus or from a reassortant virus, particularly for influenza A virus. A reassortant virus may have been obtained by reverse genetics techniques. Thus a sample may include HA from one virus strain but other influenza antigens (*e.g.* PB1, PB2, PA, NP, M1, M2, NS1 and/or NS2 proteins) from a different strain *e*.*g*. from A/PR/8/34, from A/AA/6/60, or from A/WSN/33.

The HA concentration in a sample will usually be between 0.1µg/ml and 10mg/ml *e*.*g*. between 1µg/ml and 1mg/ml, or between 10µg/ml and 100µg/ml. In some embodiments the concentration is about 30µg/ml, about 15µg/ml or about 7.5µg/ml. The invention can be used to quantify HA in this range.

The sample may include serum components but is preferably free from such components.

The sample may include egg proteins (*e.g.* ovalbumin and ovomucoid) and/or chicken DNA, but in some embodiments is free from these components *e*.*g*. when virus was grown in cell culture.

The sample may include DNA *e.g.* chicken DNA or mammalian DNA (*e.g.* derived from MDCK cells, Vero cells, PER.C6 cells, *etc*.). Ideally, however, a sample contains less than 600ng of DNA per mg of HA (preferably less than 60ng, and more preferably less than 6ng).

The sample preferably includes no viral proteins except for influenza virus proteins.

The sample may include detergent *e.g.* a polyoxyethylene sorbitan ester surfactant (known as 'Tweens' *e.g.* polysorbate 80), an octoxynol (such as octoxynol-9 (Triton X-100) or -10, or t-octylphenoxypolyethoxyethanol), a cetyl trimethyl ammonium bromide ('CTAB'), or sodium deoxycholate, particularly for a split or surface antigen vaccine sample. The detergent may be present only at trace amounts *e.g.* residual from antigen manufacture. Other sample components in trace amounts could be antibiotics (*e*.*g*. neomycin, kanamycin, polymyxin B).

The invention is described herein in relation to influenza virus HA, but it will be apparent that the principles of the invention can equally be applied to further viruses and antigens.

### Downstream steps

Methods of the invention permit the measurement of HA concentration in material of interest. This material, particularly a bulk vaccine, can then be diluted to give a desired final HA concentration. Thus a method of the invention may include a further step of diluting a vaccine based on the results of HA quantification, and the invention provides a method for analysing a vaccine comprising steps of: (a) quantifying HA in the vaccine, or in a sample thereof, by a method as disclosed herein; and (b) using the results of step (a) to calculate the HA concentration in the vaccine. The invention also provides a method for providing a bulk vaccine with a desired HA concentration, comprising said steps (a) and (b), and a further step (c) using the results of step (b) to dilute the bulk vaccine to give the desired HA concentration. Unit doses of the diluted bulk vaccine can then be extracted, and so the invention provides a method for providing a vaccine for patient use, comprising said steps (a) to (c), and a further step (d) extracting one or more unit doses of vaccine from the diluted bulk, each unit dose having a desired HA content. The extracted material can be placed into a container *e.g.* a vial or syringe.

Diluted bulk can be mixed with other components, such as an adjuvant, prior to unit dose extraction. Thus the invention provides a method for providing a bulk adjuvanted vaccine comprising said steps (a) to (c), and a further step (d) mixing the diluted bulk vaccine with an adjuvant. The invention also provides a method for providing an adjuvanted vaccine for patient use, comprising said steps (a) to (d), and a further step (e) extracting one or more unit doses of vaccine from the diluted bulk, each unit dose having a desired HA content. The extracted material can be placed into a container *e.g.* a vial or syringe.

As an alternative to mixing an extracted dose with an adjuvant, the extracted dose may instead be packaged as a first kit component in combination with a second kit component, wherein the second kit component is a vaccine adjuvant. The two kit components can be combined at the time of use to give an adjuvanted vaccine. The kit allows the adjuvant and the antigen to be kept separately until the time of use (*e.g.* as in the PREPANDRIX™ product). The components are physically separate from each other within the kit, and this separation can be achieved in various ways. For instance, the two components may be in two separate containers, such as vials. The contents of the two vials can then be mixed *e.g.* by removing the contents of one vial and adding them to the other vial, or by separately removing the contents of both vials and mixing them in a third container. In one arrangement, one of the kit components is in a syringe and the other is in a container such as a vial. The syringe can be used (*e*.*g*. with a needle) to insert its contents into the second container for mixing, and the mixture can then be withdrawn into the syringe. The mixed contents of the syringe can then be administered to a patient, typically through a new sterile needle. Packing one component in a syringe eliminates the need for using a separate syringe for patient administration. In another arrangement, the two kit components are held together but separately in the same syringe *e.g.* a dual-chamber syringe [30]. When the syringe is actuated (*e.g.* during administration to a patient) the contents of the two chambers are mixed. This arrangement avoids the need for a separate mixing step at the time of use.

Whether antigen and adjuvant are mixed during manufacture or at the time of delivery to a patient, antigen will generally be aqueous and so the mixing step involves mixing two liquids. The volume ratio of the two liquids for mixing can vary (*e*.*g*. between 5:1 and 1:5) but is generally about 1:1. Thus two kit components may include substantially the same volume of liquid as each other.

The adjuvant can enhance the immune responses (humoral and/or cellular) elicited in a patient who receives the composition. A typical adjuvant for this purpose is an oil-in-water emulsion. Various suitable emulsions are known, and they typically include at least one oil and at least one surfactant, with the oil(s) and surfactant(s) being biodegradable (metabolisable) and biocompatible. The oil droplets in the emulsion are generally less than 5µm in diameter, and advantageously the emulsion comprises oil droplets with a sub-micron diameter, with these small sizes being achieved with a microfluidiser to provide stable emulsions. Droplets with a size less than 220nm are preferred as they can be subjected to filter sterilization.

The emulsion can include oils from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used *e.g.* obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolisable and may therefore be used in the practice of this invention. The procedures for separation, purification, saponification and other means necessary for obtaining pure oils from animal sources are well known in the art. Most fish contain metabolisable oils which may be readily recovered. For example, cod liver oil, shark liver oils, and whale oil such as spermaceti exemplify several of the fish oils which may be used herein. A number of branched chain oils are synthesised biochemically in 5-carbon isoprene units and are generally referred to as terpenoids. Shark liver oil contains a branched, unsaturated terpenoid known as squalene, 2,6,10,15,19,23-hexamethyl-2,6,10,14,18,22-tetracosahexaene. Other preferred oils are the tocopherols, including DL-α-tocopherol. Emulsions comprising squalene are particularly preferred. Mixtures of oils can be used.

Surfactants can be classified by their 'HLB' (hydrophile/lipophile balance). Preferred surfactants of the invention have a HLB of at least 10, preferably at least 15, and more preferably at least 16. Examples of suitable surfactants include, but are not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Preferred surfactants for including in the emulsion are Tween 80 (polyoxyethylene sorbitan monooleate; polysorbate 80), Span 85 (sorbitan trioleate), lecithin and Triton X-100. Inclusion of polysorbate 80 is preferred.

Mixtures of surfactants can be used *e*.*g*. Tween 80/Span 85 mixtures. A combination of a polyoxyethylene sorbitan ester such as polyoxyethylene sorbitan monooleate (Tween 80) and an octoxynol such as t-octylphenoxypolyethoxyethanol (Triton X-100) is also suitable. Another useful combination comprises laureth 9 plus a polyoxyethylene sorbitan ester and/or an octoxynol.

Preferred amounts of surfactants (% by weight) are: polyoxyethylene sorbitan esters (such as Tween 80) 0.01 to 1%, in particular about 0.1 %; octyl- or nonylphenoxy polyoxyethanols (such as Triton X-100, or other detergents in the Triton series) 0.001 to 0.1 %, in particular 0.005 to 0.02%; polyoxyethylene ethers (such as laureth 9) 0.1 to 20 %, preferably 0.1 to 10 % and in particular 0.1 to 1 % or about 0.5%.

Specific oil-in-water emulsion adjuvants useful with the invention include, but are not limited to:
- A submicron emulsion of squalene, Tween 80, and Span 85. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' [31-33], as described in more detail in Chapter 10 of ref. 34 and chapter 12 of ref. 35. The MF59 emulsion advantageously includes citrate ions *e.g.* 10mM sodium citrate buffer.
- An emulsion comprising squalene, a tocopherol, and polysorbate 80. The emulsion may include phosphate buffered saline. These emulsions may have from 2 to 10% squalene, from 2 to 10% tocopherol and from 0.3 to 3% polysorbate 80, and the weight ratio of squalene:tocopherol is preferably ≤1 (*e*.*g*. 0.90) as this can provide a more stable emulsion. Squalene and polysorbate 80 may be present volume ratio of about 5:2 or at a weight ratio of about 11:5. Thus the three components (squalene, tocopherol, polysorbate 80) may be present at a weight ratio of 1068:1186:485 or around 55:61:25. One such emulsion ('AS03') can be made by dissolving Tween 80 in PBS to give a 2% solution, then mixing 90ml of this solution with a mixture of (5g of DL-α-tocopherol and 5ml squalene), then microfluidising the mixture. The resulting emulsion may have submicron oil droplets *e.g.* with an average diameter of between 100 and 250nm, preferably about 180nm. The emulsion may also include a 3-de-O-acylated monophosphoryl lipid A (3d-MPL). Another useful emulsion of this type may comprise, per human dose, 0.5-10 mg squalene, 0.5-11 mg tocopherol, and 0.1-4 mg polysorbate 80 [36] *e*.*g*. in the ratios discussed above.
- An emulsion of squalene, a tocopherol, and a Triton detergent (*e.g.* Triton X-100). The emulsion may also include a 3d-MPL (see below). The emulsion may contain a phosphate buffer.
- An emulsion comprising a polysorbate (*e.g.* polysorbate 80), a Triton detergent (*e.g.* Triton X-100) and a tocopherol (*e.g.* an α-tocopherol succinate). The emulsion may include these three components at a mass ratio of about 75:11:10 (*e.g.* 750µg/ml polysorbate 80, 110µg/ml Triton X-100 and 100µg/ml α-tocopherol succinate), and these concentrations should include any contribution of these components from antigens. The emulsion may also include squalene. The emulsion may also include a 3d-MPL (see below). The aqueous phase may contain a phosphate buffer.
- An emulsion of squalane, polysorbate 80 and poloxamer 401 ("Pluronic™ L121"). The emulsion can be formulated in phosphate buffered saline, pH 7.4. This emulsion is a useful delivery vehicle for muramyl dipeptides, and has been used with threonyl-MDP in the "SAF-1" adjuvant [37] (0.05-1% Thr-MDP, 5% squalane, 2.5% Pluronic L121 and 0.2% polysorbate 80). It can also be used without the Thr-MDP, as in the "AF" adjuvant [38] (5% squalane, 1.25% Pluronic L121 and 0.2% polysorbate 80). Microfluidisation is preferred.
- An emulsion comprising squalene, an aqueous solvent, a polyoxyethylene alkyl ether hydrophilic nonionic surfactant (*e.g.* polyoxyethylene (12) cetostearyl ether) and a hydrophobic nonionic surfactant (*e.g.* a sorbitan ester or mannide ester, such as sorbitan monoleate or 'Span 80'). The emulsion is preferably thermoreversible and/or has at least 90% of the oil droplets (by volume) with a size less than 200 nm [39]. The emulsion may also include one or more of: alditol; a cryoprotective agent (*e.g.* a sugar, such as dodecylmaltoside and/or sucrose); and/or an alkylpolyglycoside. Such emulsions may be lyophilised.
- An emulsion having from 0.5-50% of an oil, 0.1-10% of a phospholipid, and 0.05-5% of a non-ionic surfactant. As described in reference 40, preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.
- A submicron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in reference 41, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
- An emulsion comprising a mineral oil, a non-ionic lipophilic ethoxylated fatty alcohol, and a non-ionic hydrophilic surfactant (*e.g.* an ethoxylated fatty alcohol and/or polyoxyethylene-polyoxypropylene block copolymer) [42].
- An emulsion comprising a mineral oil, a non-ionic hydrophilic ethoxylated fatty alcohol, and a non-ionic lipophilic surfactant (*e.g.* an ethoxylated fatty alcohol and/or polyoxyethylene-polyoxypropylene block copolymer) [42].
- An emulsion in which a saponin (*e.g.* QuilA or QS21) and a sterol (*e.g.* a cholesterol) are associated as helical micelles [43].

The importance of measuring HA content in vaccines, and of diluting bulk vaccines to a desired HA content, arises because inactivated influenza vaccines are standardised by their HA levels. Current vaccines typically contain about 30µg/ml of HA per strain, although lower doses are also used *e.g.* for children, or in emergency situations. Fractional doses such as ½ (*i.e.* 15µg/ml HA, as in FOCETRIA™), ¼ (*i.e.* 7.5µg/ml, as in PREPANDRIX™ when administered) and ¹/₈ have been used [44,45], as have higher doses (*e.g.* 3x or 9x doses [46,47]).Thus dilution may be performed to give a HA concentration between 0.1 and 200µg/ml per influenza virus strain, preferably between 1 and 150µg/ml *e.g.* 1-90µg/ml, 1-20µgml, 0.1-15µg/ml, 0.1-10µg/ml, 0.1-7.5µg/ml, 0.5-5µg/ml, 3.75-15µg/ml *etc.* Particular post-dilution concentrations include *e.g.* about 90µg/ml, about 45µg/ml, about 30 µg/ml, about 15 µg/ml, about 10 µg/ml, about 7.5 µg/ml, about 5 µg/ml, about 3.8 µg/ml, about 3.75 µg/ml, about 1.9 µg/ml, about 1.5 µg/ml, *etc.* Lower concentrations (*e.g.* <30µg/ml) are most useful when an adjuvant is present in the vaccine. Although concentrations as high as 180µg/ml have been used in some studies (*e.g.* reference 48), compositions of the invention will usually have a HA concentration of ≤30µg/ml.

### Pharmaceutical compositions

HA-containing compositions for administration to patients are pharmaceutically acceptable. They usually include components in addition to the HA and optional adjuvant *e.g.* they typically include one or more pharmaceutical carrier(s) and/or excipient(s). A thorough discussion of such components is available in reference 49.

Pharmaceutical compositions will generally be in aqueous form *(e.g.* for injection) but solid dosage forms can also be used, and are known for influenza vaccination.

A pharmaceutical composition may include a preservative such as thiomersal (*e.g* at 10µg/ml) or 2-phenoxyethanol. It is preferred, however, that a vaccine should be substantially free from (*i.e.* less than 5µg/ml) mercurial material *e.g.* thiomersal-free [50]. Vaccines containing no mercury are more preferred. Preservative-free vaccines are particularly preferred.

To control tonicity, an aqueous pharmaceutical composition may include a physiological salt, such as a sodium salt. Sodium chloride (NaCl) is preferred, which may be present at between 1 and 20 mg/ml. Other salts that may be present include potassium chloride, potassium dihydrogen phosphate, disodium phosphate dehydrate, magnesium chloride, calcium chloride, *etc.*

Aqueous pharmaceutical compositions will generally have an osmolality of between 200 mOsm/kg and 400 mOsm/kg, preferably between 240-360 mOsm/kg, and will more preferably fall within the range of 290-310 mOsm/kg. Osmolality has previously been reported not to have an impact on pain caused by vaccination [51], but keeping osmolality in this range is nevertheless preferred.

Pharmaceutical compositions may include one or more buffers. Typical buffers include: a phosphate buffer; a Tris buffer; a borate buffer; a succinate buffer; a histidine buffer; or a citrate buffer. Buffers will typically be included in the 5-20mM range. The buffer may be in an emulsion's aqueous phase.

The pH of a pharmaceutical composition will generally be between 5.0 and 8.1, and more typically between 6.0 and 8.0 *e.g.* 6.5 and 7.5, or between 7.0 and 7.8. A method of the invention may therefore include a step of adjusting the pH of a bulk prior to dose extraction or packaging.

A pharmaceutical composition is preferably sterile. A pharmaceutical composition is preferably gluten free.

Preferred pharmaceutical compositions have a low endotoxin content *e.g.* less than 1 IU/ml, and preferably less than 0.5 IU/ml. The international unit for endotoxin measurement is well known and can be calculated for a sample by, for instance, comparison to an international standard [52,53], such as the *2nd International Standard* (Code 94/580 - IS) available from the NIBSC. Current vaccines prepared from virus grown in eggs have endotoxin levels in the region of 0.5-5 IU/ml.

A pharmaceutical composition may be free from antibiotics (*e.g.* neomycin, kanamycin, polymyxin B).

A pharmaceutical composition may include material for a single immunisation, or may include material for multiple immunisations *(i.e.* a 'multidose' composition, in which case an extracted unit dose contains enough material for more than one patient dose). Multidose arrangements usually include a preservative in the vaccine. To avoid this need, a vaccine may be contained in a container having an aseptic adaptor for removal of material.

Influenza vaccines are typically administered by intramuscular injection in a dosage volume of about 0.5ml, although a half dose (*i.e.* about 0.25ml) may be administered to children, and unit doses will be selected accordingly *e.g.* a unit dose to give a 0.5ml dose for administration to a single patient. Lower dosage volumes may be used for *e.g.* a 0.1ml volume is useful for intradermal injection.

### Packaging of compositions or kit components

Methods of the invention can include a step in which vaccine is placed into a container, and in particular into a container for distribution for use by physicians. This step will usually involve extraction of material from a bulk and its insertion into the container.

Suitable containers for aqueous vaccines include vials, nasal sprays and disposable syringes, which should be sterile.

Where a composition/component is located in a vial, the vial is preferably made of a glass or plastic material. The vial is preferably sterilised before the composition is added to it. To avoid problems with latex-sensitive patients, vials may be sealed with a latex-free stopper, and the absence of latex in all packaging material is preferred. The vial may include a single dose of vaccine, or it may include more than one dose (a 'multidose' vial) *e.g.* 10 doses. Preferred vials are made of colorless glass.

A vial can have a cap (*e.g.* a Luer lock) adapted such that a syringe can be inserted therein. A vial may have a cap that permits aseptic removal of its contents, particularly for multidose vials.

Where a composition/component is packaged into a syringe, the syringe may have a needle attached to it. If a needle is not attached, a separate needle may be supplied with the syringe for assembly and use. Such a needle may be sheathed. Safety needles are preferred. 1-inch 23-gauge, 1-inch 25-gauge and 5/8-inch 25-gauge needles are typical. Syringes may be provided with peel-off labels on which the lot number, influenza season and expiration date of the contents may be printed, to facilitate record keeping. The plunger in the syringe preferably has a stopper to prevent the plunger from being accidentally removed during aspiration. The syringes may have a latex rubber cap and/or plunger. Disposable syringes contain a single dose of vaccine. The syringe will generally have a tip cap to seal the tip prior to attachment of a needle, and the tip cap is preferably made of a butyl rubber. If the syringe and needle are packaged separately then the needle is preferably fitted with a butyl rubber shield. Useful syringes are those marketed under the trade name "Tip-Lok"™. Other useful syringes include those suitable for intradermal administration *e.g.* a microinjection device with a needle about 1.5mm long.

Containers may be marked to show a half-dose volume *e.g.* to facilitate delivery to children. For instance, a syringe containing a 0.5ml dose may have a mark showing a 0.25ml volume.

Where a glass container (*e.g.* a syringe or a vial) is used, then it is preferred to use a container made from a borosilicate glass rather than from a soda lime glass.

A composition may be combined (*e.g.* in the same box) with a leaflet including details of the vaccine *e.g.* instructions for administration, details of the antigens within the vaccine, *etc.* The instructions may also contain warnings *e.g.* to keep a solution of adrenaline readily available in case of anaphylactic reaction following vaccination, *etc.*

### Methods of treatment, and administration of the vaccine

Compositions of the invention are suitable for administration to animals, such as humans, and the invention provides a method of raising an immune response in an animal, comprising the step of administering a composition of the invention to the patient.

The invention also provides a kit or composition of the invention for use as a medicament *e.g.* for raising an immune response in an animal. The invention also provides the use of a composition of the invention in the manufacture of a medicament for raising an immune response in an animal.

Immune response raised by methods and uses of the invention will generally include an antibody response, preferably a protective antibody response. Methods for assessing antibody responses, neutralising capability and protection after influenza virus vaccination are well known in the art. Human studies have shown that antibody titers against HA of human influenza virus are correlated with protection (a serum sample hemagglutination-inhibition titer of about 30-40 gives around 50% protection from infection by a homologous virus) [54]. Antibody responses are typically measured by hemagglutination inhibition, by microneutralisation, by single radial immunodiffusion (SRID), and/or by single radial hemolysis (SRH). These assay techniques are well known in the art.

Influenza vaccines can be administered in various ways. The most preferred immunisation route is by intramuscular injection (*e.g.* into the arm or leg), but other available routes include subcutaneous injection, intranasal [55-57], intradermal [58,59], oral [60], transcutaneous, transdermal [61], *etc.* Intradermal and intranasal routes are attractive. Intradermal administration may involve a microinjection device *e.g.* with a needle about 1.5mm long.

Vaccines prepared according to the invention may be used to treat both children and adults. Influenza vaccines are currently recommended for use in pediatric and adult immunisation, from the age of 6 months. Thus the patient maybe less than 1 year old, 1-5 years old, 5-15 years old, 15-55 years old, or at least 55 years old. Preferred patients for receiving the vaccines are the elderly (*e.g.* ≥50 years old, ≥60 years old, and preferably ≥65 years), the young (*e.g.* ≤5 years old), hospitalised patients, healthcare workers, armed service and military personnel, pregnant women, the chronically ill, immunodeficient patients, patients who have taken an antiviral compound (*e.g.* an oseltamivir or zanamivir compound; see below) in the 7 days prior to receiving the vaccine, people with egg allergies and people travelling abroad. The vaccines are not suitable solely for these groups, however, and may be used more generally in a population.

Preferred compositions of the invention satisfy 1, 2 or 3 of the CPMP criteria for efficacy. In adults (18-60 years), these criteria are: (1) ≥70% seroprotection; (2) ≥40% seroconversion; and/or (3) a GMT increase of ≥2.5-fold. In elderly (>60 years), these criteria are: (1) ≥60% seroprotection; (2) ≥30% seroconversion; and/or (3) a GMT increase of ≥2-fold. These criteria are based on open label studies with at least 50 patients. The criteria apply for each strain in a vaccine.

Treatment can be by a single dose schedule or a multiple dose schedule. Multiple doses may be used in a primary immunisation schedule and/or in a booster immunisation schedule. In a multiple dose schedule the various doses may be given by the same or different routes *e.g.* a parenteral prime and mucosal boost, a mucosal prime and parenteral boost, *etc.* Administration of more than one dose (typically two doses) is particularly useful in immunologically naïve patients *e.g.* for people who have never received an influenza vaccine before, or for vaccinating against a new HA subtype. Multiple doses will typically be administered at least 1 week apart (*e.g*. about 2 weeks, about 3 weeks, about 4 weeks, about 6 weeks, about 8 weeks, about 12 weeks, about 16 weeks, *etc*.).

Vaccines produced by the invention may be administered to patients at substantially the same time as (*e.g.* during the same medical consultation or visit to a healthcare professional or vaccination centre) other vaccines *e.g.* at substantially the same time as a measles vaccine, a mumps vaccine, a rubella vaccine, a MMR vaccine, a varicella vaccine, a MMRV vaccine, a diphtheria vaccine, a tetanus vaccine, a pertussis vaccine, a DTP vaccine, a conjugated *H.influenzae* type b vaccine, an inactivated poliovirus vaccine, a hepatitis B virus vaccine, a meningococcal conjugate vaccine (such as a tetravalent A-C-W135-Y vaccine), a respiratory syncytial virus vaccine, a pneumococcal conjugate vaccine, *etc.* Administration at substantially the same time as a pneumococcal vaccine and/or a meningococcal vaccine is particularly useful in elderly patients.

Similarly, vaccines of the invention may be administered to patients at substantially the same time as (*e.g.* during the same medical consultation or visit to a healthcare professional) an antiviral compound, and in particular an antiviral compound active against influenza virus (*e.g.* an oseltamivir and/or a zanamivir).

### General

The term "comprising" encompasses "including" as well as "consisting" *e.g.* a composition "comprising" X may consist exclusively of X or may include something additional *e.g.* X + Y.

The word "substantially" does not exclude "completely" *e.g.* a composition which is "substantially free" from Y may be completely free from Y. Where necessary, the word "substantially" may be omitted from the definition of the invention.

The term "about" in relation to a numerical value x is optional and means, for example, *x*±10%.

"GI" numbering is used above. A GI number, or "GenInfo Identifier", is a series of digits assigned consecutively to each sequence record processed by NCBI when sequences are added to its databases. The GI number bears no resemblance to the accession number of the sequence record. When a sequence is updated (*e.g.* for correction, or to add more annotation or information) then it receives a new GI number. Thus the sequence associated with a given GI number is never changed.

Unless specifically stated, a method comprising a step of mixing two or more components does not require any specific order of mixing. Thus components can be mixed in any order. Where there are three components then two components can be combined with each other, and then the combination may be combined with the third component, *etc.*

Where animal (and particularly bovine) materials are used in the culture of cells, they should be obtained from sources that are free from transmissible spongiform encaphalopathies (TSEs), and in particular free from bovine spongiform encephalopathy (BSE). Overall, it is preferred to culture cells in the total absence of animal-derived materials.

Where a cell substrate is used for reassortment or reverse genetics procedures, it is preferably one that has been approved for use in human vaccine production *e.g.* as in Ph Eur general chapter 5.2.3.

Identity between polypeptide sequences is preferably determined by the Smith-Waterman homology search algorithm as implemented in the MPSRCH program (Oxford Molecular), using an affine gap search with parameters *gap open penalty*=*12* and *gap extension penalty*=*1.*

### BRIEF DESCRIPTION OF THE DRAWINGS

Figure 1 illustrates an ELISA of the invention, with GNA (triangles) immobilised on the surface of a support, binding to HA (star), to which biotinylated anti-HA IgG is then added.
Figure 2 shows a chromatogram of material purified by affinity chromatography using immobilised GNA with A/Brisbane/59/07 vaccine. The boxed fractions in the peak were pooled and analysed by SDS-PAGE, as shown in the marked lanes in Figure 3.
Figure 3 shows SDS-PAGE analysis of the fractions obtained from affinity chromatography using immobilised GNA with A/Brisbane/59/07 vaccine (as indicated in Figure 2). Left-hand 3 arrows: reduced and boiled material; right-hand 3 arrows: non-reduced non-boiled material.
Figure 4 shows OD₄₀₅ₙₘ using anti-B/Florida IgG (solid line) or anti-Solomon IgG (dotted line). The x-axis shows antigen dilution.
Figure 5 is like Figure 4, but shows analysis using IgG from sheep antisera produced by immunization with egg-produced HA of (5A) ABrisbane/59/07 H1N1 or (5B) A/Uruguay/716/2007 H3N2. Samples were H1N1 NIBSC antigen standard (■), H3N2 NIBSC antigen standard (●), or H1N1 monovalent bulk from viruses grown in cell culture (▲).
Figure 6 is like Figure 5, except the analysis of different samples is shown. Analysis using IgG from sheep antisera produced by immunization with egg-produced HA of (6A) A/Brisbane/59/07 H1N1 or (6B) A/Uruguay/716/2007 H3N2 are shown. Samples were H1N1 CBER antigen standard (■), H3N2 CBER antigen standard (●), or H3N2 monovalent bulk from viruses grown in cell culture (▲).
Figure 7 is like Figures 4 and 5, but shows analysis for monovalent bulk vaccine prepared from viral strains having low HA glycosylation: (7A) a H5N1 strain; (7B) a H1N1 strain.

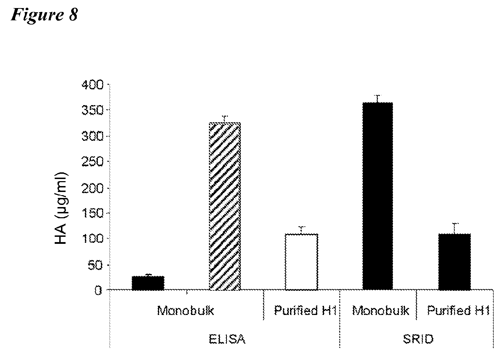

Figure 8 shows the HA concentration of samples obtained by ELISA and SRID using sheep antiserum against egg-produced A/Brisbane/59/07 (H1N1). The antigen samples were MDCK-produced A/Brisbane/59/07 monobulk and GNA-column purified HA from the monobulk. Black bars represent the concentration obtained when the egg-produced A/Brisbane/59/07 NIBSC standard reference was used as calibration standards; the striped bar indicates the concentration obtained when the purified MDCK-produced A/Brisbane/59/07 H1 was used as calibration standards; and the white bars represent the concentration obtained when the A/Brisbane/59/07 (H1N1) monobulk was used as calibration standards.

### MODES FOR CARRYING OUT THE INVENTION

### ELISA format

An ELISA of the invention can easily be prepared. Standard protocols for preparing an ELISA assay can be used, but the capture reagent on the ELISA plate is a lectin such as GNA lectin. 100µl of influenza virus vaccine is applied to each well (in duplicate) and is incubated at 37°C for 30 minutes. Each well then receives 100µl of biotinylated anti-HA IgG, followed by a further 30 minutes of incubation. The plate is then washed 4 times, and 200µl streptavidin-conjugated alkaline phosphatase is added. The plate is incubated for 30 minutes at room temperature in the dark and is then again washed 4 times. 200 µl of pNPP is then added and the enzymatic reaction is allowed to proceed for 30 minutes. Each well is then analysed to provide quantitative results. The overall process takes less than 3 hours, whereas a SRID assay takes at least 24 hours. Moreover, it can easily be performed by laboratory staff and does not require any treatment of the sample (*e.g.* no requirement for concentration, deglycosylation, denaturation, *etc*.).

The IgG used in the assay were purified from the sheep antisera provided by NIBSC or CBER using a G-protein column (HiTrap™ Protein G HP, GE Healthcare) following manufacturer instructions. IgG content in eluted fractions was determined by absorbance at 280 nm, considering that an absorbance of 1.4 OD indicates a 1 mg/ml IgG concentration. Purified fractions with OD>0.3 were pooled and dialyzed at +4°C overnight, with 4 liters of PBS 1x in a Slide-A-Lyzer Dialysis Cassettes, 10K MWCO (Pierce). During dialysis buffer was changed 3 times. The IgGs were concentrated at 1mg/ml by centrifugation at 3000 rpm using an AMICON ULTRA-15 Ultracel-10k column, then incubated 1 hour at room temperature with shaking with a 10x molar excess of NHS-PEG4-Biotin dissolved in PBS at a concentration of 3 mg/ml. After incubation IgGs were run down a PD-10 column equilibrated in PBS-2 mM EDTA (pH=7.3). Eight fractions of 1 ml were collected and the IgG biotin concentration was determined by the absorbance at 280 nm.

The ELISA plates were prepared on EIA/RIA 1x8 Stripwcll plate by adding 200 µl/well of GNA lectin at a concentration of 50 µg/ml in Ca²⁺/Mg² free PBS. Plates were wrapped in plastic foil and incubated overnight at +20°C. Wells were then aspirated and washed 4 times with 350 µl/well of wash buffer (PBS Ca²⁺/Mg²⁺ 1x - Tween 20 0.05%, pH 7.4). Then plates were incubated with 350µl/well of blocking buffer (Tris-HCl 10 mM - NaCl 150 mM - Sucrose 3% - BSA 1%, pH 7.68) for 1 hour at room temperature. Blocking buffer was aspirated and the plates could be immediately used for the assay or dried overnight at room temperature in a lyophilizer and then stored at +4°C in a heated sealed foil pouch with a desiccant pack.

For performing the sandwich ELISA seven 2-fold serial dilutions of the samples were made in Ca²⁺/Mg²⁺-free PBS. Each dilution was loaded in duplicates on GNA-coated well (100 µl/well) and incubated 30 minutes at 37°C with shaking. After the incubation, biotinylated-IgGs (diluted at 8 (µg/ml in biotin diluent (50mM Tris - 150mM NaCl - 0.05% Tween20 - 20% lamb serum, pH 7.5) were added (100 µl/well) to the antigen without washing and incubated for other 30 minutes at 37°C with shaking. Wells were then aspirated and washed 4 times with 350 µl/well of wash buffer (50mM Tris - 150 mM NaCl - 0.05% Tween 20) with an automatic plate washer. IgG binding was then detected by adding 200 µl/well of streptavidin-conjugated alkaline phosphatase diluted at 1 µg/ml in wash buffer, for 30 minutes at 37°C with shaking. At the end of this step the plate was aspirated and washed as before, then 200 µl/well of pNPP was added to the wells and incubated 30 minutes at room temperature. Plates were read at 405nm in a microplate reader.

### ELISA specificity

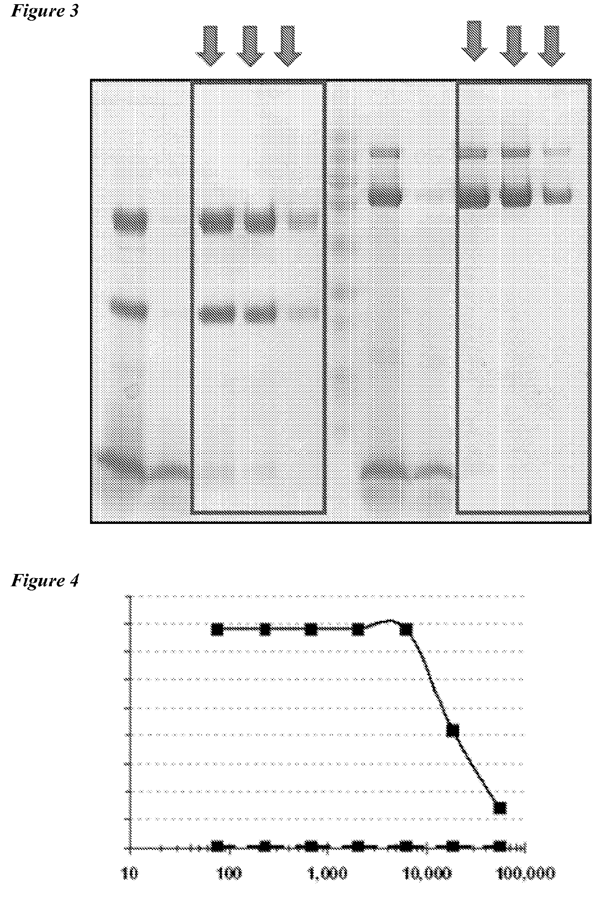

To confirm strain specificity the ELISA was tested using captured B/Florida/4/2006 antigen prepared from with IgG either for that strain or for the unrelated A/Solomon/3/2006 strain. Figure 4 shows OD₄₀₅ₙₘ for monovalent vaccine bulk prepared from MDCK cells (Optaflu bulk) against antigen dilution. The anti-B/Florida IgG reliably detected the B/Florida HA (solid line) but the anti-Solomon IgG did not (dotted line).

Similarly, IgG against A/Brisbane/59/07 (II1N1) or Uruguay/716/2007 (II3N2) were tested against the relevant reference antigens from NIBSC, and against monovalent Optaflu bulks for these strains.

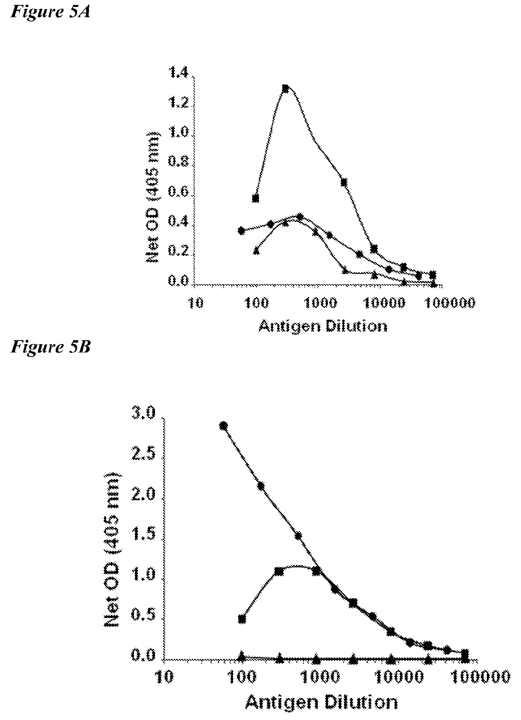

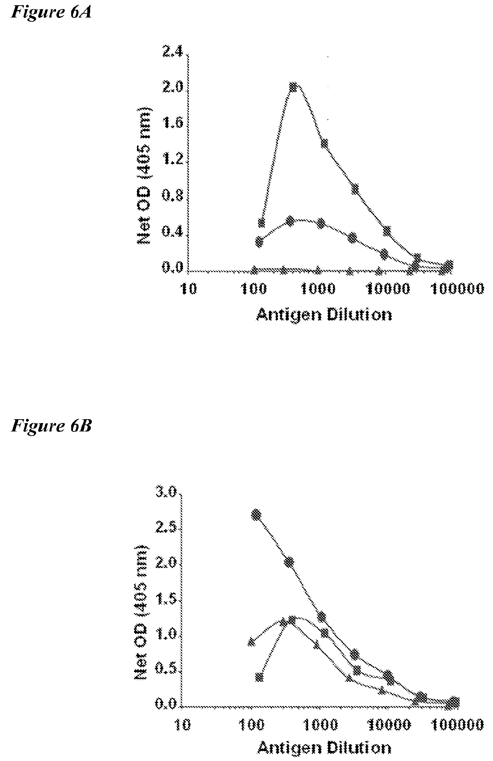

Figures 5 and 6 confirm specificity of the ELISA. H1N1 or H3N2 MDCK-produced monobulks were detected only by the IgG specific for each strain; the egg-produced CBER or NIBSC antigen standards generated positive ELISA signals with either the H1N1- or H3N2-specific antisera.

The most likely explanation for the cross-reactivity observed when sheep antisera raised against egg-produced HA were used to detect egg-produced antigen samples (but not when used to detect MDCK cell-produced antigen samples) is the presence of egg-derived proteins in the egg-produced antigen samples and anti-egg protein antibodies in the sheep antisera. This conclusion is corroborated by western blots (not shown) which indicate the cross reactive antibody recognizes proteins that do not co-migrate with HA.

### Broad strain applicability

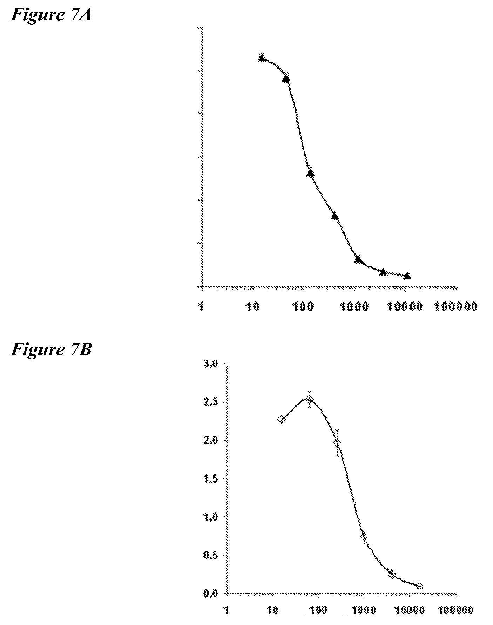

To confirm that the ELISA method is broadly suitable for influenza virus strains the GNA lectin capture was tested with monovalent bulk vaccine prepared from strains having low glycosylation, namely A/Vietnam/1194/2004 (H5N1) and A/California/04/2009 (H1N1). Figure 7 shows that the capture works well even with these strains. Thus the capture lectin is easily adaptable to different strains, independent of their HA glycosylation levels.

### Comparison with SRID

Samples were taken at 13 stages during production of a monovalent A/Brisbane/59/07 (H1N1) bulk vaccine. These were analysed for HA content by SRID, by RP-HPLC, or by the ELISA of the invention. The SRID and RP-HPLC assays were calibrated using the relevant NIBSC standard; the ELISA was calibrated either with purified A/Brisbane/59/07 hemagglutinin or with SRID-calibrated A/Brisbane/59/07 monovalent bulk. HA concentration in the ELISA was calculated as the mean of at least three different dilutions (coefficient of variation≤20%).

Results at each stage were as follows (µg/ml; '-' = not detectable):

| **Stage** | **SRID** | **RP-HPLC** | **ELISA¹** | **ELISA²** |
|---|---|---|---|---|
| 1 | - | 34 | 19 | 19 |
| 2 | - | 33 | 16 | 16 |
| 3 | - | 32 | 16 | 16 |
| 4 | - | 31 | 18 | 19 |
| 5 | - | 3 | 2 | 2 |
| 6 | - | 2 | - | - |
| 7 | - | 2 | 4 | 4 |
| 8 | - | 2 | - | - |
| 9 | 342 | 313 | 280 | 328 |
| 10 | 124 | 138 | 97 | 125 |
| 11 | 180 | 175 | 142 | 176 |
| 12 | 129 | 127 | 106 | 130 |
| 13 | 29 | 19 | 36 | 36 |

| | | | | |
|---|---|---|---|---|
| 1: ELISA reference was purified protein; 2: ELISA reference was SRID-calibrated monobulk | | | | |

Thus the ELISA was able to quantify HA even at levels too low for detection by SRID, and the quantitative results are in line with those measured by other techniques.

### Preparative HA purification

An affinity support with immobilised GNA lectin was obtained from Vector Laboratories Inc in Burlingame California (catalog #AL-1243), and was used with a GE Healthcare HR 16/10 (20ml) column. Monovalent bulk influenza vaccines were mixed with n-Octyl-beta-D-glucopyranoside (octylglucoside, OG; final concentration 1.5%) and incubated on ice. This material was applied to a washed column, and then eluted using α-methyl-mannopyranoside.

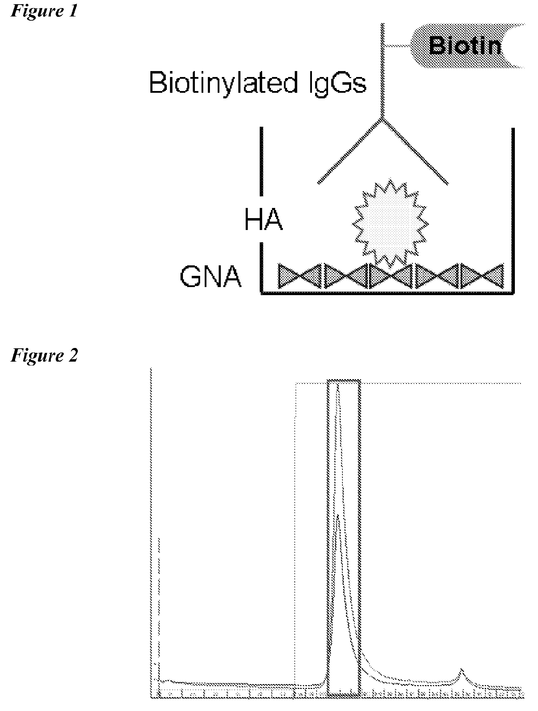

Figure 2 shows a chromatogram of material purified by affinity chromatography using immobilised GNA lectin with A/Brisbane/59/07 vaccine. The boxed fractions were pooled and dialysed against PBS. The dialysed material was analysed by SDS-PAGE (Figure 3). The HA is very pure and so GNA lectin affinity chromatography can be used for preparative purification of HA.

### Antigen standards for the HA ELISA

Figure 8 shows that when an NIBSC antigen standard and antiserum (both egg-platform based) are used to calibrate the ELISA format of the invention for detection of HA in an MDCK-produced vaccine monobulk, the HA content of the monobulk is grossly underestimated (27 µg/ml, first column from the left) relative to the content determined using the same reagents in SRID (365 µg/ml, fourth column from the left). Thus, it appears that the relative contributions of anti-HA and anti-host (egg) antibody-antigen interactions to the signal in ELISA and SRID are different. The inventors consider that, because the precipitin ring in SRID results from the antibody interactions of primarily the most abundant antigen in the sample (HA), host cell protein and anti-host cell protein antibody effects may be much less important in SRID than in ELISA.

The inventors hypothesized that using purified HA as an antigen standard would eliminate host cell protein effects on the ELISA, bringing its results into alignment with those obtained by SRID. To test this hypothesis, the inventors used HA produced in MDCK cells, purified on a GNA-column, and quantified by a conventional SRID at 109 µg/ml (Figure 8, first column from the right) using egg-based antigen standard and antiserum as the standard for the ELISA. The quantity of the purified HA determined by conventional SRID corresponded well to the quantity of HA in this sample determined by physical methods. Absorption at 280 nm indicated 100 µg/ml HA and BCA assay indicated 83 µg/ml HA. Using this purified HA standard brought the ELISA value for the MDCK-produced monobulk (327 µg/ml, second column from the left in Figure 8) into reasonable correspondence with the SRID value for the monobulk (365 µg/ml).

The hypothesis that matrix effects in the ELISA, but not in SRID, are responsible for discrepancies between ELISA and SRID predicts that an MDCK-produced monobulk, quantified by SRID with egg-based antigen standard and antiserum could be used to calibrate the ELISA for use with MDCK-produced samples, provided that the sheep antiserum used for detection in the ELISA was elicited by immunization with egg-produced HA. Consistent with this hypothesis, the quantity of MDCK-produced purified HA by ELISA (100 µg/ml, third column from the left in Figure 8) calibrated with this monobulk standard matched the quantity of the same purified HA sample, determined by standard SRID (109 µg/ml). Therefore, the data suggest that cell derived monobulk quantified with standard SRID-assigned HA concentrations can be used as standard for ELISA to quantify MDCK-produced antigens.

In conclusion, the ELISA is sensitive to anti-host (for example, egg) antibodies in conventional antisera used for SRID. Therefore, the data suggest that it is best to use serum raised against egg-produced HA to detect mammalian cell-produced HA. Antigen calibration standards for the ELISA format of the invention should be purified or at least should not be produced from the same platform that produced the antigen used to immunize the sheep to avoid such anti-host cell protein effects.It will be understood that the invention has been described by way of example only and modifications may be made whilst remaining within the scope and spirit of the invention.

### REFERENCES

[1] Williams (1993) Vet Microbiol 37:253-262.
[2] Fitzgerald & Needy (1986) Dev Biol Stand 64:73-79.
[3] WO2010/136896.
[4] WO2005/090390.
[5] Li et al. (2010) Biologicals 38:284-9.
[6] Brady & Griffiths (1982) J Biol Standardization 10:9-15.
[7] Mahmood & Hay (1992) J Immunol Meth 151:9-13.
[8] Li et al. (2009) Curr Chem Viol 3:324-33.
[9] Wolff and Reichl (2011) Expert Rev. Vaccines 10:1451-1475.
[10] WO2007/066231.
[11] Opitz et al. (2007) Vaccine 939-947
[12] Optiz et al. (2008) Journal of Virological Methods 154:61-68
[13] Opitz et al. (2007) Journal of Biotechnology 131:309-317
[14] WO96/37624.
[15] WO98/46262.
[16] WO95/18861.
[17] Bright et al. (2008) PLoS ONE 3:e1501.
[18] Crevar & Ross (2008) Virology Journal 5:131.
[19] Vaccines. (eds. Plotkin & Orenstein). 4th edition, 2004, ISBN: 0-7216-9688-0.
[20] Huckriede et al. (2003) Methods Enzymol 373:74-91.
[21] GenBank sequence GI:325176.
[22] WO2008/032219
[23] WO97/37000.
[24] Brands et al. (1999) Dev Biol Stand 98:93-100.
[25] Halperin et al. (2002) Vaccine 20:1240-7.
[26] Tree et al. (2001) Vaccine 19:3444-50.
[27] EP-A-1260581 (WO01/64846).
[28] WO2006/071563.
[29] WO2005/113758.
[30] WO2008/001221.
[31] WO90/14837.
[32] Podda & Del Giudice (2003) Expert Rev Vaccines 2:197-203.
[33] Podda (2001) Vaccine 19: 2673-2680.
[34] Vaccine Design: The Subunit and Adjuvant Approach (eds. Powell & Newman) Plenum Press 1995 (ISBN 0-306-44867-X).
[35] Vaccine Adjuoants: Preparation Methods and Research Protocols (Volume 42 of Methods in Molecular Medicine series). ISBN: 1-59259-083-7. Ed. O'Hagan.
[36] WO2008/043774.
[37] Allison & Byars (1992) Res Immunol 143:519-25.
[38] Hariharan et al. (1995) Cancer Res 55:3486-9.
[39] US-2007/014805.
[40] WO95/11700.
[41] US patent 6,080,725.
[42] WO2006/113373.
[43] WO2005/097181.
[44] WO01/22992.
[45] Hehme et al. (2004) Virus Res. 103(1-2):163-71.
[46] Treanor et al. (1996) J Infect Dis 173:1467-70.
[47] Keitel et al. (1996) Clin Diagn Lab Immunol 3:507-10.
[48] Zangwill et al. (2008) J Infect Dis. 197(4):580-3.
[49] Gennaro (2000) Remington: The Science and Practice of Pharmacy. 20th edition, ISBN: 0683306472.
[50] Banzhoff (2000) Immunology Letters 71:91-96.
[51] Nony et al. (2001) Vaccine 27:3645-51.
[52] Poole & Mussett (1989) JBiol Stand 17:161-71.
[53] Poole et al. (1997) J. Endotoxin Res 4:221-31
[54] Potter & Oxford (1979) Br Med Bull 35: 69-75.
[55] Greenbaum et al. (2004) Vaccine 22:2566-77.
[56] Zurbriggen et al. (2003) Expert Rev Vaccines 2:295-304.
[57] Piascik (2003) J am Pharm Assoc (Wash DC). 43:728-30.
[58] Halperin et al. (1979) Am JPublic Health 69:1247-50.
[59] Herbert et al. (1979) J Infect Dis 140:234-8.
[60] Mann et al. (2004) Vaccine 22:2425-9.
[61] Chen et al. (2003) Vaccine 21:2830-6.

## Claims

1. A method for detecting influenza virus hemagglutinin in a sample, comprising steps of: (i) contacting the sample with a lectin which is immobilised on a solid support, to provide captured hemagglutinin; and (ii) detecting the captured hemagglutinin by immunoassay.

2. The method of claim 1, wherein the lectin is a GNA lectin.

3. The method of any preceding claim, wherein step (ii) is a quantitative step which permits determination of the amount and/or concentration of hemagglutinin in the sample.

4. The method of any preceding claim, wherein the sample contains (a) hemagglutinin from only one influenza virus strain or (b) hemagglutinin from multiple influenza virus strains.

5. The method of any preceding claim, wherein the HA comprises influenza A virus HA.

6. The method of claim 5, wherein the HA is H1, H3 or H5 HA.

7. The method of any preceding claim, wherein the HA comprises influenza B virus HA.

8. The method of any preceding claim, wherein HA in the sample is from an influenza virion.

9. The method of any preceding claim, wherein the sample includes (i) influenza virus hemagglutinin and (ii) influenza virus antigens PB1, PB2, PA, NP, NA, M1, M2, NS1 and/or NS2.

10. The method of any preceding claim, wherein the sample includes whole influenza virions.

11. The method of any preceding claim, wherein the sample includes split influenza virions.

12. The method of any preceding claim, wherein the sample includes purified surface antigens from influenza virions.

13. The method of any preceding claim, wherein the HA is HA1.

14. The method of any preceding claim, wherein the sample is free from (i) serum components, (ii) egg proteins, and/or (iii) chicken DNA.

15. The method of any preceding claim, wherein the sample includes no viral proteins except for influenza virus proteins.

16. The method of any preceding claim, wherein the sample includes detergent.

17. The method of any preceding claim, wherein the sample is a bulk vaccine.

18. A method for analysing a vaccine comprising steps of: (a) detecting hemagglutinin in the vaccine, or in a sample thereof, by the method of any preceding claim; and (b) using the results of step (a) to calculate the hemagglutinin concentration in the vaccine.

19. A method for providing a bulk vaccine with a desired hemagglutinin concentration, comprising steps of: (a) analysing hemagglutinin in the vaccine, or in a sample thereof, by the method of claim 18; (b) using the results of step (a) to calculate the hemagglutinin concentration in the bulk vaccine; and (c) using the results of step (b) to dilute the bulk vaccine to give the desired hemagglutinin concentration.

20. The method of claim 19, wherein the desired hemagglutinin concentration is between 1 and 150µg/ml *e*.*g*. 90µg/ml, 45µg/ml, 30 µg/ml, 15 µg/ml, 10 µg/ml, 7.5 µg/ml, 5 µg/ml, 3.8 µg/ml, 3.75 µg/ml, 1.9 µg/ml, or 1.5 µg/ml.

21. A method for providing a vaccine for patient use, comprising steps of: preparing a bulk vaccine with a desired hemagglutinin concentration by the method of claim 19 or claim 20; and then extracting one or more unit doses of vaccine from the diluted bulk.

22. The method of claim 21, wherein an extracted unit dose is packaged as a kit component in combination with a second kit component, wherein the second kit component is a vaccine adjuvant.

23. A method for providing a bulk adjuvanted vaccine comprising steps of: preparing a bulk vaccine with a desired hemagglutinin content by the method of claim 19 or claim 20; and then mixing the diluted bulk vaccine with an adjuvant.

24. A method for providing an adjuvanted vaccine for patient use, comprising steps of: providing a bulk adjuvanted vaccine by the method of claim 23; and then extracting one or more unit doses of vaccine from the bulk.

25. An ELISA kit comprising (i) a multi-well solid support (ii) a lectin and (iii) an enzymatic influenza virus hemagglutinin detection reagent.

26. An ELISA method for quantifying influenza virus hemagglutinin in a sample, comprising steps of: (i) contacting the sample with a lectin which is immobilised on a solid support, to provide captured hemagglutinin; (ii) attaching an enzyme to the captured hemagglutinin via an anti-hemagglutinin antibody; and (iii) using the attached enzyme to convert a substrate to a product, wherein quantitative detection of the product indicates the quantity of influenza virus hemagglutinin in the sample.

27. A method for purifying influenza virus hemagglutinin in a sample, comprising a step of contacting the sample with a lectin which is immobilised on a solid support, wherein the lectin is a mannose-binding lectin, with the proviso that the lectin is not a ConA lectin.

28. The method of claim 27, wherein the lectin is a GNA lectin.


SEQUENCE LISTING

<110>   Novartis AG

<120>   Assays for influenza virus hemagglutinins

<130>   P060225WO

<141>   2012-12-12

<150>   US 61/569,597
<151>   2011-12-12

<160>   6

<170>   SeqWin2010, version 1.0

<210>   1
<211>   157
<212>   PRT
<213>   Galanthus nivalis

<400>   1
Met Ala Lys Ala Ser Leu Leu Ile Leu Ala Thr Ile Phe Leu Gly Val
1               5                   10                  15

Ile Thr Pro Ser Cys Leu Ser Glu Asn Ile Leu Tyr Ser Gly Glu Thr
            20                  25                  30

Leu Pro Thr Gly Gly Phe Leu Ser Ser Gly Ser Phe Val Phe Ile Met
        35                  40                  45

Gln Glu Asp Cys Asn Leu Val Leu Tyr Asn Val Asp Lys Pro Ile Trp
    50                  55                  60

Ala Thr Asn Thr Gly Gly Leu Ser Ser Asp Cys Ser Leu Ser Met Gln
65                  70                  75                  80

Asn Asp Gly Asn Leu Val Val Phe Thr Pro Ser Asn Lys Pro Ile Trp
                85                  90                  95

Ala Ser Asn Thr Asp Gly Gln Asn Gly Asn Tyr Val Cys Ile Leu Gln
            100                 105                 110

Lys Asp Arg Asn Val Val Ile Tyr Gly Thr Asn Arg Trp Ala Thr Gly
        115                 120                 125

Thr Tyr Thr Gly Ala Val Gly Ile Pro Glu Ser Pro Pro Ser Glu Lys
    130                 135                 140

Tyr Pro Ser Ala Gly Lys Ile Lys Leu Val Thr Ala Lys
145                 150                 155

<210>   2
<211>   157
<212>   PRT
<213>   Galanthus nivalis

<400>   2
Met Ala Lys Ala Ser Leu Leu Ile Leu Thr Thr Ile Phe Leu Gly Val
1               5                   10                  15

Ile Thr Pro Ser Cys Leu Ser Glu Asn Ile Leu Tyr Ser Gly Glu Thr

20                  25                  30

Leu Pro Thr Gly Gly Ser Leu Thr Ser Gly Ser Phe Val Phe Ile Met
        35                  40                  45

Gln Gln Asp Cys Asn Leu Val Leu Tyr Asn Val Asp Lys Pro Ile Trp
    50                  55                  60

Ala Thr Asn Thr Gly Gly Leu Ser Ser Asp Cys Ser Ile Ser Leu Gln
65                  70                  75                  80

Thr Asp Gly Asp Leu Val Val Tyr Thr Pro Ser Asn Lys Pro Ile Trp
                85                  90                  95

Ala Ser Asn Thr Asp Gly Gln Asn Gly Asn Tyr Val Cys Ile Leu Gln
            100                 105                 110

Lys Asp Arg Asn Val Val Ile Tyr Gly Thr Asn Arg Trp Ala Thr Gly
        115                 120                 125

Thr Tyr Thr Gly Ala Val Gly Ile Pro Glu Ser Pro Pro Ser Glu Lys
    130                 135                 140

Tyr Pro Thr Ala Gly Met Ile Lys Leu Val Thr Ala Lys
145                 150                 155

<210>   3
<211>   157
<212>   PRT
<213>   Galanthus nivalis

<400>   3
Met Ala Lys Ala Ser Leu Leu Ile Leu Ala Thr Ile Phe Leu Gly Val
1               5                   10                  15

Ile Thr Pro Ala Cys Leu Ser Glu Asn Ile Leu Tyr Ser Gly Glu Thr
            20                  25                  30

Leu Pro Thr Gly Gly Phe Leu Thr Ser Gly Ser Phe Val Phe Ile Met
        35                  40                  45

Gln Glu Asp Cys Asn Leu Val Leu Tyr Asn Val Asp Lys Pro Ile Trp
    50                  55                  60

Ala Thr Asn Thr Gly Gly Leu Ser Ser Asp Cys Ser Leu Ser Met Gln
65                  70                  75                  80

Thr Asp Gly Asn Leu Val Val Tyr Thr Pro Ser Asp Lys Pro Ile Trp
                85                  90                  95

Ala Ser Asp Thr Asp Gly Gln Asn Gly Asn Tyr Val Cys Ile His Gln
            100                 105                 110

Lys Asp Arg Asn Val Val Ile Tyr Gly Thr Asn Arg Trp Ala Thr Gly
        115                 120                 125

Thr His Thr Gly Ala Val Gly Ile Pro Ala Ser Pro Pro Ser Glu Lys
    130                 135                 140

Tyr Pro Thr Ala Gly Met Ile Lys Leu Val Thr Ala Lys
145                 150                 155

<210>   4

<211>   157
<212>   PRT
<213>   Galanthus nivalis

<400>   4
Met Ala Lys Ala Ser Leu Leu Ile Leu Ala Thr Ile Phe Leu Gly Val
1               5                   10                  15

Ile Thr Pro Ser Cys Leu Ser Glu Asn Ile Leu Tyr Ser Gly Glu Thr
            20                  25                  30

Leu Pro Thr Gly Gly Phe Leu Thr Ser Gly Ser Phe Val Phe Ile Met
        35                  40                  45

Gln Gln Asp Cys Asn Leu Val Leu Tyr Asn Ile Asp Lys Pro Ile Trp
    50                  55                  60

Ala Thr Asn Thr Cys Gly Leu Ser Ser Asp Cys Ser Leu Ser Met Gln
65                  70                  75                  80

Thr Asp Gly Asn Leu Val Val Tyr Thr Pro Ser Asn Lys Pro Ile Trp
                85                  90                  95

Ala Ser Asn Thr Asp Gly Gln Asn Gly Asn Tyr Val Cys Ile Leu Gln
            100                 105                 110

Lys Asp Arg Asn Val Val Ile Tyr Gly Thr Asn Arg Trp Ala Thr Gly
        115                 120                 125

Thr His Thr Gly Ala Val Gly Ile Leu Ala Ser Pro Pro Ser Glu Lys
    130                 135                 140

Tyr Pro Thr Ala Gly Met Ile Lys Leu Val Thr Ala Lys
145                 150                 155

<210>   5
<211>   157
<212>   PRT
<213>   Galanthus nivalis

<400>   5
Met Ala Lys Ala Ser Leu Leu Ile Leu Ala Thr Ile Phe Leu Gly Val
1               5                   10                  15

Ile Thr Pro Ala Cys Leu Ser Glu Asn Ile Leu Tyr Ser Gly Glu Thr
            20                  25                  30

Leu Pro Thr Gly Gly Phe Leu Thr Ser Gly Ser Phe Val Phe Ile Met
        35                  40                  45

Gln Gln Asp Cys Asn Leu Val Leu Tyr Asn Ile Asp Lys Pro Ile Trp
    50                  55                  60

Ala Thr Asn Thr Gly Gly Leu Ser Ser Asp Cys Ser Leu Ser Met Gln
65                  70                  75                  80

Thr Asp Gly Asn Leu Val Val Tyr Asn Pro Ser Asn Lys Pro Ile Trp
                85                  90                  95

Ala Ser Asn Thr Asp Gly Gln Asn Gly Asn Tyr Val Cys Ile Leu Gln
            100                 105                 110

Lys Asp Arg Asn Val Val Ile Tyr Gly Thr Asn Arg Trp Ala Thr Gly

        115                 120                 125

Thr His Thr Gly Ala Val Gly Ile Leu Ala Ser Pro Pro Ser Glu Lys
    130                 135                 140

Tyr Pro Thr Ala Gly Met Ile Lys Leu Val Thr Ala Lys
145                 150                 155

<210> 6
<211> 160
<212> PRT
<213> Galanthus nivalis

<400> 6
Met Ala Lys Ala Ser Leu Leu Ile Leu Ala Ala Ile Phe Leu Gly Val
1               5                   10                  15

Ile Thr Pro Ser Ser Pro Ser Cys Leu Gly Asp Asn Ile Leu Tyr Ser
            20                  25                  30

Gly Glu Thr Leu Ser Thr Gly Glu Phe Leu Asn Tyr Gly Gly Phe Val
        35                  40                  45

Phe Ile Met Gln Glu Asp Cys Asn Leu Val Leu Tyr Asp Val Asp Lys
    50                  55                  60

Pro Ile Trp Ala Thr Asn Thr Gly Gly Leu Ser Arg Ser Cys Phe Leu
65                  70                  75                  80

Ser Met Gln Thr Asp Gly Asn Leu Val Val Tyr Thr Pro Ser Asn Lys
                85                  90                  95

Pro Ile Trp Ala Ser Asn Thr Gly Gly Gln Asn Gly Asn Tyr Val Cys
            100                 105                 110

Ile Leu Gln Lys Asp Arg Asn Val Val Ile Tyr Gly Thr Asp Arg Trp
        115                 120                 125

Ala Thr Gly Thr Arg Thr Gly Ala Val Gly Ile Pro Ala Ser Pro Pro
    130                 135                 140

Ser Glu Lys Tyr Pro Thr Ala Gly Lys Ile Lys Leu Val Thr Ala Lys
145                 150                 155                 160